Europäisches Patentamt

European Patent Office

Office européen des brevets

(11) Publication number: 0 276 150
A2

# EUROPEAN PATENT APPLICATION

(21) Application number: 88300474.9

(22) Date of filing: 21.01.88

(51) Int. Cl.⁴: A 61 M 5/32

(30) Priority: 21.01.87 GB 8701230

(43) Date of publication of application:
27.07.88 Bulletin 88/30

(84) Designated Contracting States:
AT BE CH DE ES FR GR IT LI LU NL SE

(71) Applicant: Burke, Nigel Francis Patrick
15 Sherdley Road
Crumpsall Green Manchester (GB)

(72) Inventor: Burke, Nigel Francis Patrick
15 Sherdley Road
Crumpsall Green Manchester (GB)

(74) Representative: Barker, Rosemary Anne et al
Barlow, Gillett & Percival 94 Market Street
Manchester M1 1PJ (GB)

(54) Syringe disposal aid.

(57) A syringe disposal aid 10 comprises a tube-like container 12 having one rounded closed end 14 and one open end 16 closed by a screw threaded removable lid or cap 18. The rounded end 14 has a slot 20 therein, the slot 20 having a relatively wide section 22 and a relatively narrow section 24, for engagement with a common syringe arrangement 26. The slot's dimensions are chosen so that a needle 34 and attached sheath 32 may be inserted into the container 12 via the wide section 22 of the slot 20. Slideable displacement of the syringe 26 causes engagement of the sheath's flange 36 with the narrow section 24 of the slot 20 thus retaining the needle 34 within the container on withdrawal of the syringe therefrom.

# Description

## SYRINGE DISPOSAL AID

This invention relates to a syringe disposal aid for the safe handling and disposal of hypodermic syringe needles.

The handling and subsequent disposal of used hypodermic syringe needles is the source of a health risk to the user. This risk arises from the possibility of puncture wounds to the user's fingers and consequent infection by foreign matter present on the needle. In normal practice this possibility of a puncture wound is caused by the detachment of the syringe needle from its syringe barrel by use of the fingers. A further possible risk arises during the storing of the needles which are usually placed in a cardboard container for subsequent disposal. If bare, i.e. uncovered, needles are placed in such a container there is a possibility that one or more needles may puncture the container and hence be a possible source of infection.

The problems identified in the previous paragraph can be partially overcome by re-sheathing the needle in its container cap. Unfortunately the cap is not designed for this purpose and the re-sheathing operation is a further source of a wound to the user's fingers. In view of possible infection by a lethal blood contaminant for example HIV or hepatitis organisms the handling of a used needle in such a manner is dangerous to the user's health.

It is an object of the present invention to provide a syringe disposal aid which will permit the safe removal of a syringe needle from its barrel and the safe storage of the needle prior to its destruction.

With this object in view the present invention provides a syringe disposal aid comprising a rigid container having a fitting disposed so as to communicate with the container's interior and shaped so as to be engageable with a syringe needle's flange to allow the needle to be separated from its barrel and accommodated within the container.

Preferably the container has a removable lid so as to permit access to the container interior.

Advantageously the fitting is a slot in the container shaped so as to allow the insertion of a syringe needle into the container and to permit engagement of the syringe needle's flange with a part of the slot so that the needle can be detached for storing in the container.

Preferably the container is provided with a radial collar to prevent the needle slipping into a user's hand.

Advantageously the container is provided with a base or mounting means so that it is unnecessary for its user to hold it.

Preferably the container may contain a germicide or absorbent compound to prevent accidental escape of contaminated material.

Advantageously a part or all of the container is transparent so that its contents can be viewed.

Preferably the container is shaped so as to prevent it rolling or other involuntary movements.

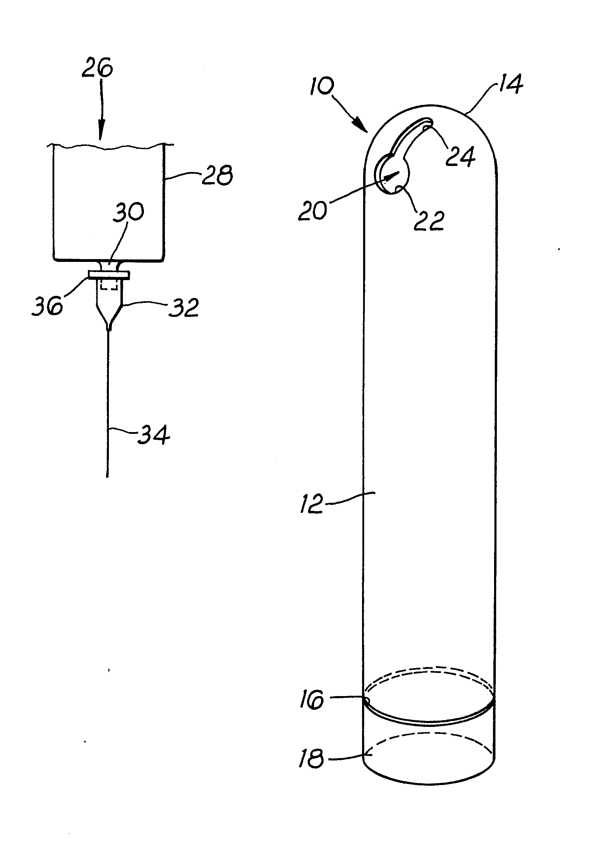

The invention will be described further by way of example with reference to the accompanying drawing in which the single figure shows a preferred embodiment of a syringe disposal aid according to the invention.

A preferred embodiment of a syringe disposal aid is illustrated in the single figure and is referred to generally by the numeral 10. The syringe disposal aid 10 comprises a container 12 of generally cylindrical configuration with one rounded closed end 14 and having its opposite end 16 closed by a screw threaded cap or lid 18. A fitting in the form of a slot 20 is provided at the rounded end 14, the slot 20 having a one section of relatively large width 22 and a relatively narrow section 24.

The shape of the slot 20 is adapted to engage with a standard syringe arrangement 26 including a syringe barrel or body 28 having a nipple 30. Fitted over the nipple 30 is a sheath 32 to which is attached a hollow syringe needle 34. The sheath 32 is usually provided with a flange 36 to facilitate engagement with the nipple 30. The slot 20 is thus shaped so that the sheath 32 and flange 36 are able to be inserted into the container 12 through the wide section 22 of the slot 20. The narrow section 24 of the slot 20 has a width sufficient to allow the nipple 30 to be inserted therein but not the flange 36.

In use a syringe 26 having a used and possibly contaminated needle 34 is inserted into the container 12 through the wide section 22 of slot 20. The syringe 26 is then slideably displaced along the slot 20 into the relatively narrow section 24 where the slots edges engage with the nipple 30. Withdrawal of the syringe 26 will cause the flange 34 to catch on the slots edges and hence be pulled off the nipple 30 and to be retained in the container 12. Additional needles may be placed into the container as necessary.

The invention is not confined to the foregoing details and variations may be made thereto. For example the container is preferably of metal although any rigid and puncture proof material could be used e.g. a rigid plastic. The slot 20 may be provided with a cap or cover to seal the tube between use. A radial collar may be provided around the body of the container below the slot to prevent a needle, which has accidentally missed being inserted into the slot, from wounding the user. A base or mounting means may be provided to ensure the container is secure during use. The container can contain a germicide or similar bacteriological agent for partially decontaminating the needle or alternatively an absorbent material. At least part or all of the tube may be transparent so that its contents may be viewed. A corrosive material may be placed in the container to ensure that any needles placed therein are rendered unusable. The container may be of any desired shape although a tube is preferred which may be slightly or partially flattened to limit rolling. Other possible shapes for the container are square or hexagonal cross-sectional tubes, boxes or any other convenient shape. The screw threaded cap or lid could be omitted or be secured by other means.

Other variations may also be possible.

## Claims

1. A syringe disposal aid comprising a rigid container having a fitting disposed so as to communicate with the containers interior and shaped so as to be engageable with a syringe needle's flange to allow the needle to be separated from its barrel and to be enclosed within the container.

2. A syringe disposal aid as claimed in claim 1 wherein the container has a removable lid.

3. A syringe disposal aid as claimed in claims 1 or 2 wherein the fitting is a slot in the container shaped so as to allow the insertion of a syringe needle into the container and to permit engagement of the syringe needle's flange with a part of the slot so that the needle can be detached.

4. A syringe disposal aid as claimed in claims 1, 2 or 3 wherein the container is provided with a radial collar to prevent a needle slipping into a user's hand.

5. A syringe disposal aid as claimed in any preceding claims wherein the container is provided with mounting means for attachment to a fixed surface.

6. A syringe disposal aid as claimed in any preceding claim wherein at least a part of the container is transparent.

7. A syringe disposal aid as claimed in any preceding claim wherein the container is shaped to prevent accidental movement thereof.

8. A syringe disposal aid substantially as hereinbefore described with reference to and as illustrated in the accompanying drawings.

0276150